(19) Europäisches Patentamt
European Patent Office
Office européen des brevets

(11) Veröffentlichungsnummer: **0 062 291**
**B1**

# (12) EUROPÄISCHE PATENTSCHRIFT

(45) Veröffentlichungstag der Patentschrift: **28.03.84**

(51) Int. Cl.³: **C 07 C 45/74,** C 07 C 49/203

(21) Anmeldenummer: **82102701.8**

(22) Anmeldetag: **31.03.82**

(54) **Verbessertes Verfahren zur Herstellung mehrfach ungesättigter Ketone.**

(30) Priorität: **08.04.81 DE 3114071**

(43) Veröffentlichungstag der Anmeldung: **13.10.82 Patentblatt 82/41**

(45) Bekanntmachung des Hinweises auf die Patenterteilung: **28.03.84 Patentblatt 84/13**

(84) Benannte Vertragsstaaten: **CH DE FR GB LI NL**

(56) Entgegenhaltungen:
**DE - A - 2 637 428**
**FR - A - 2 275 430**

(73) Patentinhaber: **BASF Aktiengesellschaft, Carl-Bosch-Strasse 38, D-6700 Ludwigshafen (DE)**

(72) Erfinder: **Janitschke, Lothar, Dr., Wormser Gasse 9A, D-6711 Kleinniedesheim (DE)**
Erfinder: **Hoffmann, Werner, Dr., Ringstrasse 11 C, D-6708 Neuhofen (DE)**
Erfinder: **Arnold, Lothar, Dr., Hausackerweg 11, D-6900 Heidelberg (DE)**
Erfinder: **Stroezel, Manfred, Zur Bergstrasse 10, D-6804 Ilvesheim (DE)**
Erfinder: **Scheiper, Hans-Juergen, Pfalzring 46, D-6704 Mutterstadt (DE)**

**EP 0 062 291 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verbessertes Verfahren zur Herstellung mehrfach ungesättigter Ketone

Die Erfindung betrifft ein verbessertes Verfahren zur Herstellung von mehrfach ungesättigten Ketonen, insbesondere von den Verbindungen 6-Methyl-hepta-3,5-dien-2-on, Pseudojonon, Methylpseudojonon, Dimethylpseudojonon, Pseudoiron, Methylpseudoiron und Dimethylpseudoiron, die als Riechstoffe bzw. Riechstoffzwischenprodukte von grossem wirtschaftlichen Interesse sind. Pseudojonon ist darüber hinaus ein bedeutendes Zwischenprodukt für die technische Herstellung von Vitamin A und 6-Methylhepta-3,5-dien-2-on ein begehrter Aromastoff.

Entsprechend ihrer technischen Bedeutung hat es nicht an Versuchen gefehlt, diese Verbindungen möglichst vorteilhaft herzustellen. So sind zahllose Verfahren zur Herstellung derselben beschrieben, von denen wir hier nur die nächstliegenden erläutern möchten.

So erhält man beispielsweise nach Arosov et al. (SU 138612 von 1960) 6-Methyl-hepta-3,5-dien-2-on in 53%iger Ausbeute, wenn man ein Gemisch aus 3-Methyl-but-2-en-1-al und Wasser (wie es bei der Oxidation von 3-Methyl-but-2-en-1-ol anfällt) unter Kühlung zu einem Gemisch aus 20 g NaOH und 160 ml trockenem Aceton gibt. Die Asubeute ist für ein technisches Verfahren unbefriedigend.

Nach R. Fischer et al. (DE-OS 2150992 von 1971) gelingt die Kondensation von 3-Methyl-but-2-en-1-al mit Aceton oder Methyläthylketon (Molverhältnis ca. 1/3) zu 6-Methyl-hepta-3,5-dien-2-on bzw. 7-Methyl-octa-4,6-dien-3-on in 3 Stunden bei 180°C und 45 bar in Gegenwart von ZnO in einer Ausbeute von 93, bzw. 88% bezogen auf umgesetztes 3-Methyl-but-2-en-1-al bei einem Umsatz von 76 bzw. 68%. Nachteilig an diesem Verfahren sind lange Reaktionszeiten verbunden mit geringen Umsätzen, wodurch sich nur kleine Raum-Zeit-Ausbeuten ergeben.

Zur Herstellung von Pseudojonon aus Citral sind zahlreiche Methoden bekannt basierend auf der Kondensation von Citral mit Aceton in Anwesenheit von Basen in wässrigen oder nichtwässrigen Lösungsmitteln. Im wasserfreien Medium benutzt man als Kondensationsmittel Alkalialkoholate oder Phenolate und als Lösungsmittel Alkohol oder Benzol (Russel et al., Org. Synth. Bd. III, Seiten 380–384 und CS-PS 85207 von 1966).

Die Verwendung von Akoholaten bzw. Phenolaten erfordert deren gesonderte Herstellung und stellt hohe Anforderungen an die Trockenheit aller Reaktionsteilnehmer, was die Umsetzung sehr aufwendig macht und bei der technischen Herstellung nicht sonderlich vorteilhaft ist.

Diese Nachteile vermeiden Verfahren zur Kondensation von Citral mit Aceton in wässrigen Lösungsmitteln, bei denen man als Kondensationsmittel Sulfite (vgl. DDR 28759 von 1960) bzw. Alkalihydroxide einsetzt. Nachteilig an dem Verfahren in Gegenwart von Sulfiten sind unbefriedigende Ausbeuten an Pseudojonon sowie das Auftreten schwer abtrennbarer Verunreinigungen.

Die Kondensation von Citral mit Aceton in Gegenwart wässriger Lösungen von Basen stellt eine billigere Variante dieses Verfahrens dar. Zur Kondensation von Citral mit Aceton in Anwesenheit wässriger Lösungen von Basen sind mehrere Verfahren bekannt, die sich im Verhältnis der Komponenten, in der Temperatur und/oder in der Reaktionsdauer unterscheiden. Wir wollen uns in der Beschreibung auf die bisher vorteilhaftesten Verfahren beschränken. So ist aus Chem. Abstract 71 (1969) P 38354x die Umsetzung von Citral mit einem grossen Überschuss an Aceton in Gegenwart stark verdünnter wässriger Natronlauge bei 30 bis 40°C bekannt. Man erhält bei diesem Verfahren das Pseudojonon in 1,5 Stunden bei 40°C in einer Ausbeute von 90%. Nachteilig an diesem Verfahren ist, dass man zur Unterdrükkung unerwünschter Nebenreaktionen mit extrem stark verdünntem Reaktionsmedium arbeiten muss, wodurch einerseits die erzielten Raum-Zeit-Ausbeuten nur sehr gering sind und andererseits sowohl der Energieaufwand bei der Verdampfung des Acetons aus dem sich unter den gegebenen Bedingungen nicht in Phasen trennenden Reaktionsgemisch als auch der Aufwand bei der Extraktion aus den sehr verdünnten Lösungen extrem hoch sind.

Gemäss der SU-PS 704938 von 1978 wird zur Vermeidung der beschriebenen Nachteile vorgeschlagen, die Umsetzung mit einem 15- bis 20fachen Acetonüberschuss und mit einem Volumenverhältnis von Aceton:Wasser im Reaktionsgemisch von 1:0,15 bis 1:0,45 durchzuführen. Die hierbei erzielten Ausbeuten sind gut. Nachteilig an diesem Verfahren ist vor allem die extrem lange Prozessdauer von 2,5 bis 5 Stunden, wodurch die erzielten Raum-Zeit-Ausbeuten sehr gering werden.

Gemäss der SU-PS 546603 von 1974 wird vorgeschlagen, die basenkatalysierte Umsetzung von Citral mit Aceton oder Methyläthylketon in Anwesenheit von Kondensationsprodukten von Citral mit Citral oder von Citral mit dem entsprechenden Keton in Mengen von 20 bis 100%, bezogen auf eingesetztes Citral, durchzuführen. Auch bei diesem Verfahren sind die erzielten Ausbeuten recht gut. Nachteilig an diesem Verfahren ist wiederum die lange Reaktionsdauer. Beispielsweise wird in Beispiel 2 nach einer Zulaufszeit von 2 Stunden noch 3 Stunden erwämrt. Hierdurch ergeben sich nur unbefriedigende Raum-Zeit-Ausbeuten.

Es war die Aufgabe der Erfindung, das Verfahren zur Herstellung der ungesättigten Ketone der allgemeinen Formel I durch Umsetzen von α,β-ungesättigten Aldehyden mit Aceton oder Methyläthylketon in Gegenwart wässriger Alkalilauge derart zu verbessern, dass man die ungesättigten Ketone unter Vermeidung der Nachteile der bekannten Verfahren, d.h. auf einfache Weise in guten Ausbeuten bei gleichzeitigen guten Raum-Zeit-Ausbeuten erhält.

Gegenstand der Erfindung ist ein Verfahren zur Herstellung ungesättigter Ketone der allgemeinen Formeln Ia und/oder Ib

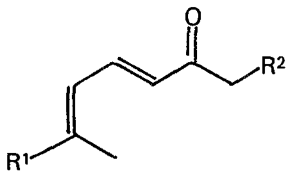
(I a)

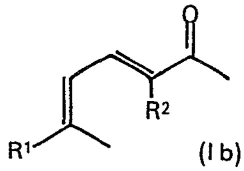
(I b)

in welcher der Rest $R^1$ folgende Bedeutung haben kann:

$-CH_3$;

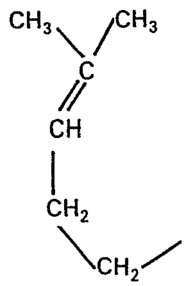

oder

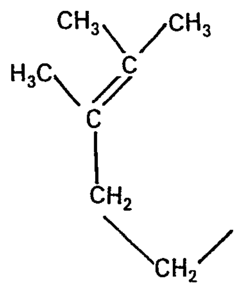

und der Rest $R^2$ für $-H$ oder $-CH_3$ steht, durch Umsetzen eines Aldehyds der allgemeinen Formel II

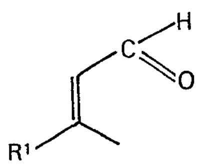
(II)

mit einem molaren Überschuss eines Ketons der allgemeinen Formel III

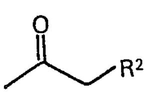
(III)

bei erhöhter Temperatur in Gegenwart einer wässrigen Alkalilauge, das dadurch gekennzeichnet ist, dass man

a) die Reaktion unter so intensiver Durchmischung aller Reaktionspartner vornimmt, dass sich das zweiphasige Reaktionsgemisch nicht entmischt und der Durchmesser der Tropfen im Reaktionsgemisch gleich oder kleiner als 1 mm ist,

b) hierbei eine Temperatur einhält, die 10 bis 120°C, vorzugsweise 25 bis 75°C, oberhalb des Siedepunktes bei Normaldruck der niedrigstsiedenden Reaktionskomponente liegt

c) einen Druck von p bis 100 bar einhält, wobei p der Dampfdruck des Reaktionsgemisches bei der Reaktionstemperatur ist, und

d) das Keton der Formel III in Mengen von 8 bis 30 Mol pro Mol Aldehyd der Formel II einsetzt.

Es war sehr überraschend, dass sich die Raum-Zeit-Ausbeuten bei der Umsetzung der empfindlichen α,β-ungesättigten Aldehyde II mit Aceton oder Methyläthylketon in Gegenwart von wässriger Alkalilauge unter den erfindungsgemässen Bedingungen ausserordentlich verbessern lassen, ohne dass die Ausbeuten an ungesättigten Ketonen durch die drastischeren Bedingungen verschlechtert werden. Bisher wurde allgemein maximal unter Rückflusstemperaturen gearbeitet und die Notwendigkeit des Erwärmens bei dieser Umsetzung wegen den damit verbundenen Verlusten an Aceton und Citral und wegen der beschleunigten Verharzung des Reaktionsansatzes (vgl. SU-PS 704 938, Spalte 3, Zeilen 14–20) als Nachteil betrachtet. Die starken Vorurteile gegen höheres Erhitzen eines Reaktionsansatzes, der die Aldehyde II, das Keton III und wässrige Alkalilauge enthält, ist sehr verständlich, wenn man bedenkt, dass bekannt ist, dass α,β-ungesättigte Aldehyde, speziell in Gegenwart von Alkali, stark zu Selbstkondensationsreaktionen und Polymerisationsreaktionen neigen [vgl. z.B. H. Labbé, Bull. Soc. Chim. France 21 (1899) 407 und Thomas, Helv. Chim. Acta 59 (1976) 2261–67].

Es ist zwar aus der DE-OS 2 637 428 ein Verfahren zur Kondensation von Aldehyden mit Ketonen bekannt, bei welchem bei Verwendung von Alkalihydroxiden als Katalysatoren Reaktionstemperaturen von 20 bis 80°C als möglich bezeichnet werden, jedoch handelt es sich hierbei um die Kondensation von gesättigten oder α,β-ungesättigten Aldehyden, die in α-Stellung zur Carbonylgruppe eine Isopropylgruppe enthalten und daher weit weniger zu Selbstkondensationen und Polymerisationen in wässrig alkalischem Milieu neigen als die in α-Stellung unsubstituierten Aldehyde der Formel II gemäss der vorliegenden Erfindung. Die gemäss diesem bekannten Verfahren erzielten Ausbeuten und Raum-Zeit-Ausbeuten lassen die äusserst vorteilhaften Ergebnisse des erfindungsgemässen Verfahrens nicht erwarten.

Als Aldehyde der Formel II kommen in Betracht: 3-Methyl-2-buten-1-al, 3,7-Dimethyl-2,6-octadien-1-al (Citral) und 3,6,7-Trimethyl-2,6-octadien-1-al. Die genannten Aldehyde sind bekannte Verbindungen, die in an sich bekannter Weise hergestellt werden können.

Die verwendeten Ketone Aceton und Methyläthylketon sind handelsübliche Verbindungen. Man verwendet sie im allgemeinen in molarem Überschuss, insbesondere in Mengen von 8 bis 30, vorzugsweise 9 bis 15 Mol pro Mol Aldehyd. Geringere Mengen führen zu einer Verlängerung der Verweilzeit sowie zu Ausbeuteeinbussen.

Mengen über 30 Mol bringen keinen wirtschaftlichen Vorteil mehr. Nicht umgesetztes Keton wird bei der Aufarbeitung zurückgewonnen und kann erneut eingesetzt werden.

Als wässrige Alkalilaugen verwendet man im allgemeinen bis 0,005 bis zu 20-gew.%ige wässrige Lösungen von NaOH oder KOH. Für die Umsetzung mit Aceton bedient man sich vorzugsweise einer etwa 0,1 bis 15-gew.%igen, insbesondere einer etwa 0,3- bis 7-gew.%igen, wässrigen Alkalihydroxidlösung, für die Umsetzung mit Methyläthylketon, vorzugsweise einer 0,5- bis 20-gew.%igen Lösung.

Die Alkalihydroxide verwendet man im allgemeinen in Mengen von 0,001 bis 0,6 Mol, vorzugsweise 0,005 bis 0,5 Mol pro Mol Aldehyd.

Ausschlaggebend für die benötigte Menge an Lauge ist u.a. der Säuregehalt des eingesetzten Aldehydes.

Zur Durchführung des erfindungsgemässen Verfahrens wird ein Gemisch der Aldehyde II mit den Ketonen III und der wässrigen Alkalilauge unter intensiver Durchmischung entweder bei konstanter Reaktionstemperatur oder in einem bestimmten Temperaturintervall bei dem Reaktionsdruck umgesetzt.

Sehr wichtig für ein vorteilhaftes Gelingen der Umsetzung ist das intensive Durchmischen, damit das im allgemeinen zweiphasige Reaktionsgemisch sich nicht entmischt. Zum Durchmischen von Katalysatorlösung und Reaktionsteilnehmern sind alle hierfür üblichen und brauchbaren Vorrichtungen wie Rührkessel, statische Mischer (beispielsweise Sulzer-Mischer und Kenics-Mischer) und dynamische Mischer (beispielsweise hochdrehende Pumpen und hochdrehende Rührorgane) sowie Mischdüsen geeignet, welche ein so intensives Durchmischen ermöglichen, dass der Durchmesser der Tropfen im Reaktionsgemisch gleich oder kleiner als 1 mm, vorzugsweise gleich oder kleiner als 0,1 mm ist. Das Durchmischen des Rekationsgemisches kann vor dem Eintritt des Reaktionsgemisches in den Reaktor durch Mischdüsen oder andere Mischeinbauten erfolgen, wenn der Reaktor so dimensioniert ist, dass während der Umsetzung keine Entmischung eintritt. Der Reaktor kann aber auch so ausgestaltet sein, dass er gleichzeitig als Mischer dient. Welcher Mischer am vorteilhaftesten ist, hängt von der Art der Reaktionsführung und dem Reaktionsgefäss ab. Sehr vorteilhaft ist es, die Umsetzung so durchzuführen, dass das Reaktionsgemisch den Reaktor mit konstanter Tropfengrösse und konstanter Tropfenströmung durchläuft.

Ein weiteres wesentliches Merkmal des erfindungsgemässen Verfahrens ist, dass man bei Temperaturen oberhalb des Siedepunktes des Reaktionsgemisches bei Normaldruck und daher auch bei einem Druck oberhalb des Dampfdrukkes des Reaktionsgemisches bei der Siedetemperatur arbeitet. Praktisch handelt es sich um Temperaturen, die 10 bis 120, vorzugsweise 25 bis 75°C, oberhalb des Siedepunktes der niedrigstsiedenden Reaktionskomponente liegt. Für Umsetzungen mit Aceton liegen diese Temperaturen bei 65 bis 180, vorzugsweise bei 80 bis 130°C, für Umsetzungen mit Methyläthylketon bei 90 bis 200, vorzugsweise bei 105 bis 155°C.

Der Druck kann der Dampfdruck des Gemisches bei der Reaktionstemperatur sein, er kann aber auch bis zu 100 bar, vorzugsweise bis zu 20 bar erhöht sein. Insbesondere bei kontinuierlicher Arbeitsweise ist es vorteilhaft, wenn der Druck durch Zugabe eines Inertgases auf einen Druck oberhalb des Dampfdruckes eingestellt wird.

Zur Erzielung eines praktisch quantitativen Umsatzes genügen unter den erfindungsgemässen Bedingungen Verweilzeiten unterhalb von 45 Minuten, speziell unterhalb von 30 Minuten. Bei kontinuierlicher Arbeitsweise im Reaktionsrohr mit vorgeheizten Ausgangsstoffen sind Verweilzeiten von 4 bis 10 Minuten die Regel.

Wenn man bedenkt, dass für diese Umsetzungen nach den bekannten Verfahren Verweilzeiten von 2 bis 8 Stunden und mehr üblich sind, ergeben sich dementsprechend bei dem erfindungsgemässen Verfahren drastische Erhöhungen der Raum-Zeit-Ausbeuten gegenüber denen der Normaldruckfahrweise.

Das Verfahren lässt sich kontinuierlich oder diskontinuierlich durchführen. Besonders vorteilhaft ist die kontinuierliche Arbeitsweise. Im letzteren Fall kann man sich mehrerer hintereinandergeschalteter Reaktionsgefässe oder einer Reaktionskolonne bedienen. Bevorzugt verwendet man jedoch Rohrreaktoren mit geeigneten Abmessungen, insbesondere solche, in denen unter den Reaktionsbedingungen eine ausreichend turbulente Strömung herrscht, so dass sich das zweiphasige Reaktionsgemisch innerhalb des Reaktors nicht entmischt.

Mit besonderem Vorteil arbeitet man so, dass man zu einem vorgeheizten Gemisch aus Aldehyd II und Keton III unter den Reaktionsbedingungen die gegebenenfalls vorgeheizte Katalysatorlösung gibt. Hierbei ist die Anwendung definierter optimaler Reaktionszeiten gewährleistet.

Nach Verlassen des Kondensationsreaktors wird das Gemisch mit billigen organischen Säuren, wie Essigsäure, oder billigen anorganischen Säuren, wie Schwefelsäure neutralisiert. Im Fall der Schwefelsäure kann es vorteilhaft sein, die Konzentration der Säure geringer als 10%, insbesondere geringer als 3% zu wählen, um das Ausfallen von Natriumsulfat zu verhindern. Prinzipiell sind alle Säuren einschliesslich saurer Ionenaustauscher geeignet.

Man kann aber auch das Reaktionsgemisch nach Verlassen des Kondensationsreaktors in einem Phasentrenngefäss von der Hauptmenge der Alkalilauge befreien, bevor es mit Säuren neutralisiert wird. Die abgetrennte Alkalilauge kann dann erneut bei der Umsetzung verwendet werden.

Zur weiteren Aufarbeitung des Reaktionsgemisches wird sodann das Keton III abdestilliert, wobei vorzugsweise nur ein bestimmter Teil des Wassers mit abdestilliert wird und der Rest die bei der Neutralisation gebildeten Salze in Lösung hält. Auch diese destillative Entfernung des Ketons III gestaltet sich bei der technischen Durchführung des erfindungsgemässen Verfahrens besonders vorteilhaft, da durch die hohe Reaktortemperatur das Reaktionsgemisch bereits heiss ist und das Keton schnell verdampft.

Von dem erhaltenen Destillationsrückstand wird die wässrige salzhaltige untere Phase abgetrennt. Aus der oberen organischen Phase wird das Verfahrensprodukt durch Destillation in sehr reiner Form isoliert. Hierbei fällt der nicht umgesetzte Aldehyd II im Destillationsvorlauf an. Der zurückgewonnene Aldehyd II sowie das zurückgewonnene Keton III können erneut für die Reaktion eingesetzt werden.

Die Ausbeute an dem Keton I beträgt bis zu 95% (umgerechnet als 100%iges Keton), bezogen auf eingesetzten 100%igen Aldehyd II bzw. bis zu 100%, bezogen auf umgesetzten Aldehyd II.

Die erfindungsgemäss hergestellten Verbindungen sind interessante Riechstoffe bzw. Riechstoff-Zwischenprodukte. Das Pseudojonon ist zusätzlich eine bedeutende Zwischenstufe bei der technischen Vitamin-A-Synthese und 6-Methyl-hepta-3,5-dien-2-on ist ein begehrter Aromastoff.

Der besondere Vorteil des erfindungsgemässen Verfahrens besteht darin, dass unter den erfindungsgemässen Reaktionsbedingungen die Verweilzeit für die Reaktionsteilnehmer im Reaktor ohne Verringerung der Ausbeute – und z.T. sogar unter Erhöhung der Ausbeute – drastisch erniedrigt werden kann, wodurch sich die Raum-Zeit-Ausbeute für die Umsetzung entsprechend drastisch erhöht.

Beispiel 1

Kontinuierliche Darstellung von 6,10-Dimethyl-undeca-3,5,9-trien-2-on (Pseudojonon; Ia)

Innerhalb einer Flüssig-Flüssig-Mischdüse wurde jeweils die aus der folgenden Tabelle ersichtliche Menge einer 4,76%igen wässrigen NaOH-Lösung in einer auf 90°C vorgeheizten Mischung aus Citral (IIa) und Aceton (IIIa) Molverhältnis 1:10, Menge siehe Tabelle) dispergiert und die gut gemischte zweiphasige Flüssigkeit von oben nach unten durch einen 480 ml-Rohrreaktor (Rohr: 24 mm Durchmesser, 1200 mm Länge) mit von unten her zentral eingeführter Thermohülse gepumpt. Das Rekationsrohr wurde in der Weise gleichmässig beheizt, dass die Ofeneingangstemperatur etwa 90°C und die Ofenaustrittstemperatur etwa 110°C (genaue Werte siehe Tabelle) betrug. Der Reaktionsdruck wurde durch eine Austragsregelung auf 5 bar gehalten.

Nach Austritt aus dem Rohrreaktor wurde das Reaktionsgemsich gekühlt, auf Normaldruck entspannt und neutralisiert.

Nach Einstellen konstanter Reaktionsbedingungen wurden über jeweils 1 Stunde Zuläufe und Austräge zwecks Bestimmung der Ausbeute gemessen. Von dem neutralisierten Austrag wurde hierzu zunächst bei Normaldruck (Badtemperatur 100°C) das Aceton (wasserhaltig) abdestilliert, von dem Rückstand die Wasserphase abgetrennt und die organische Phase destilliert. Es wurden vier Fraktionen bei 0,1 bis 0,01 mbar genommen:

1) Druck: 0,1 mbar, Badtemperatur: bis 120°C; Siedebereich: bis 90°C.
2) Druck: 0,05 mbar, Badtemperatur: bis 160°C;
3) Druck: 0,05 mbar, Badtemperatur: bis 190°C;
4) Druck: 0,01 mbar, Badtemperatur: bis 240°C.

Die jeweils auf 1 Stunde bezogenen Daten sind in der folgenden Tabelle zusammengefasst.

Die Reinheit des verwendeten Citrals betrug ca. 90,2% (Beispiele a, b und f), 72,2% (Beispiel c), 88,9% (Beispiel d) sowie 87,5% (Beispiel e). In Beispiel e war der Isomerengehalt 72,0% cis- und 15,8% trans-Citral. Im Beispiel f wurde bei 60°C mit 2,5%iger Schwefelsäure und in den übrigen Beispielen mit Eisessig bei Raumtemperatur neutralisiert.

Tabelle 1

| Beispiel | | | a | b | c | d | e | f |
|---|---|---|---|---|---|---|---|---|
| Zuläufe | Citral (IIa) | [g/h] | 735,5 | 827,1 | 986,9 | 1052,4 | 1141,4 | 1217,2 |
| | | [Mol/h] | 4,36 | 4,90 | 4,68 | 6,14 | 6,56 | 7,21 |
| | Aceton (IIIa) | [g/h] | 2531,4 | 2846,9 | 2720,8 | 3569,7 | 3811,4 | 4190,2 |
| | | [Mol/h] | 43,6 | 49,0 | 46,8 | 61,4 | 65,6 | 72,1 |
| | wässr. NaOH | [g/h] | 249,7 | 433,8 | 469,8 | 568,7 | 555,5 | 1058,0 |
| | NaOH | [Mol/h] | 0,297 | 0,516 | 0,559 | 0,677 | 0,661 | 1,26 |
| Reaktionstemperatur | | [°C] | 89–109 | 90–111 | 88–108 | 90–110 | 89–110 | 103–131 |
| Verweilzeit | | [min] | 6,51 | 5,82 | 5,74 | 4,61 | 4,34 | 3,75 |
| Neutralisierter Austrag | | [g/h] | 2866,6 | 4075,0 | 4010,0 | 6350,0 | 5500 | 8000,0 |
| abdest. Aceton | | [g/h] | 2400,0 | 2640,6 | 2436,7 | 3145,0 | 2805,0 | 4428,6 |
| abgetr. Wasser | | [g/h] | 343,0 | 326,0 | 358,3 | 265,0 | 385,5 | 1371,4 |
| Organ. Rückstand | | [g/h] | 1061,0 | 1091,3 | 1177,0 | 1575,5 | 1532,5 | 2122,3 |

Tabelle 1 (Fortsetzung)

| Beispiel | | a | b | c | d | e | f |
|---|---|---|---|---|---|---|---|
| Fraktion 1 Menge | [g/h] | 155,0 | 242,7 | 203,7 | 147,5 | 164,5 | 333,7 |
| Ia | [%] | 40,8 | 63,1 | 36,6 | 49,0 | 32,4 | 54,2 |
| IIa | [%] | 15,5 | 9,6 | 16,5 | 24,2 | 21,2 | 6,3 |
| Fraktion 2 Menge | [g/h] | 635,0 | 575,3 | 658,7 | 1057,5 | 1000,0 | 989,7 |
| Ia | [%] | 98,9 | 97,9 | 95,2 | 96,2 | 95,5 | 97,8 |
| IIa | [%] | 0,2 | 0,3 | 1,1 | 1,9 | 1,4 | 0,3 |
| Fraktion 3 Menge | [g/h] | 25,0 | 42,3 | 33,3 | 33,5 | 89,0 | 69,1 |
| Ia | [%] | 98,1 | 99,9 | 89,5 | 84,4 | 79,5 | 97,2 |
| Fraktion 4 Menge | [g/h] | 24,3 | 79,3 | 74,7 | 26,5 | 24,5 | 121,7 |
| Ia | [%] | 7,2 | 26,1 | 10,5 | 12,9 | 27,2 | 20,3 |
| Rückstand | [g/h] | 158,7 | 113,3 | 86,3 | 266,5 | 200,0 | 581,1 |
| Gesamtmenge Ia (100%ig) | [Mol/h] | 3,73 | 4,05 | 3,84 | 5,83 | 5,65 | 6,45 |
| Ausbeute bez. auf einges. Citral | [%] | 85,6 | 82,7 | 82,1 | 95,0 | 86,1 | 89,5 |
| Umsatz | [%] | 95,0 | 94,3 | 94,3 | 94,0 | 95,1 | 97,8 |
| Ausbeute bez. auf umges. Citral | [%] | 90,1 | 87,7 | 87,0 | 100 | 90,5 | 91,5 |

Beispiel 2

Darstellung von 3,6,9-Trimethylundeca-3,5,9-trien-2-on (iso-Methylpseudojonon) und 7,11-Dimethyldodeca-4,6,10-trien-3-on (n-Methylpseudojonon)

22,8 g ca. 92%iges Citral (0,137 Mol) wurden zusammen mit 108 g (1,5 Mol) Methyläthylketon im Autoklaven auf 130°C erhitzt. Nach Erreichen der Reaktionstemperatur wurden 12 ml 10%ige Natronlauge zugepumpt und das Reaktionsgemisch 20 Min. bei 130° intensiv gerührt. Anschliessend wurden 40 ml einer 10%igen wässrigen Essigsäurelösung zugepumpt und der Autoklav auf Raumtemperatur abgekühlt. Nach Entspannen auf Normaldruck wurde das zweiphasige Reaktionsgemisch in 500 ml n-Hexan aufgenommen und die Wasserphase abgetrennt. Die organische Phase wurde zweimal mit je 250 ml Wasser gewaschen und mit wasserfreiem Natriumsulfat getrocknet.

Das Lösungsmittel wurde bei 20 mbar und 50°C Badtemperatur abdestilliert und der Rückstand (28,0 g) bei 200°C Badtemperatur über eine Brücke destilliert ($Kp_{0,05}$ = 56–112°C). Auf diese Weise wurden 21,8 g eines Gemisches erhalten, das nach Gaschromatogramm aus 3,6% Citral und 94,7% iso-Methylpseudojonon und n-Methylpseudojonon als Cis-trans-Isomerengemisch bestand. Die Ausbeute berechnet auf 100%iges Isomerengemisch betrug 73,1% bezogen auf 100%iges Citral, bei einem Isomerenverhältnis von ca. 15:85 = iso-Methylpseudojonon : n-Methylpseudojonon.

**Patentansprüche**

1. Verfahren zur Herstellung ungesättigter Ketone der allgemeinen Formeln Ia und/oder Ib

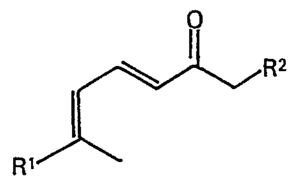

(I a)

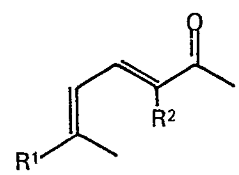

(I b)

in welcher der Rest $R^1$ folgende Bedeutung haben kann:

$-CH_3$;

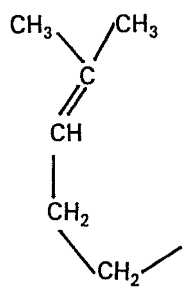

oder

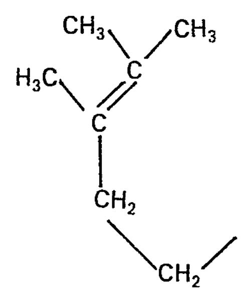

und der Rest $R^2$ für H oder $-CH_3$ steht, durch Umsetzen eines Aldehyds der allgemeinen Formel II

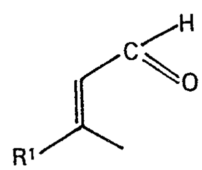

(II)

mit einem molaren Überschuss eines Ketons der allgemeinen Formel III

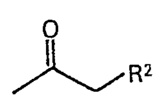

(III)

bei erhöhter Temperatur in Gegenwart einer wässrigen Alkalilauge, dadurch gekennzeichnet, dass man

a) die Reaktion unter so intensiver Durchmischung aller Reaktionspartner vornimmt, dass sich das zweiphasige Reaktionsgemisch nicht entmischt und der Durchmesser der Tropfen im Reaktionsgemisch gleich oder kleiner als 1 mm ist,
b) hierbei eine Temperatur einhält, die 10 bis 120°C oberhalb des Siedepunktes bei Normaldruck der niedrigstsiedenden Reaktionskomponente liegt,
c) einen Druck von p bis 100 bar einhält, wobei p der Dampfdruck des Reaktionsgemisches bei der Reaktionstemperatur ist, und
d) das Keton der Formel III in Mengen von 8 bis 30 Mol pro Mol Aldehyd der Formel II einsetzt.

2. Verfahren zur Herstellung ungesättigter Ketone der allgemeinen Formel I gemäss Anspruch 1, dadurch gekennzeichnet, dass man bei der Umsetzung eine Temperatur einhält, die 25 bis 75°C oberhalb des Siedepunktes der niedrigstsiedenden Reaktionskomponente liegt.

**Claims**

1. A process for the preparation of unsaturated ketones of the general formulae Ia and/or Ib

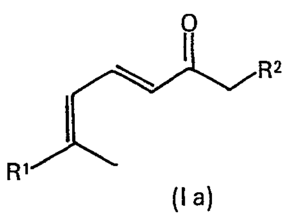
(I a)

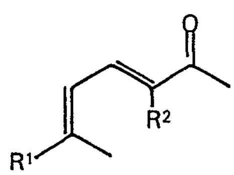
(I b)

where $R^1$ is $-CH_3$, 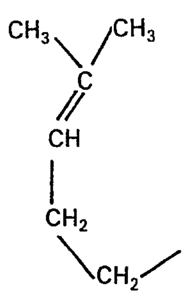 or

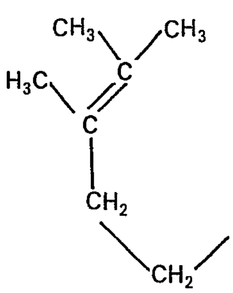

and $R^2$ is H or $-CH_3$, by reacting an aldehyde of the general formula II

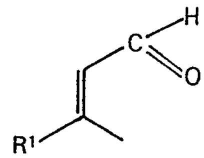
(II)

with a molar excess of a ketone of the general formula III

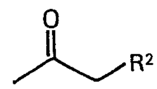
(III)

at an elevated temperature in the presence of an aqueous alkali metal hydroxide solution, wherein

a) the reaction is carried out with so intensive mixing of all reactants that the two-phase reaction mixture does not demix, and the diameter of the droplets in the reaction mixture is not more than 1 mm,
b) the temperature is kept at from 10 to 120°C above the boiling point of the lowest-boiling component at atmospheric pressure,
c) the pressure is kept at from p to 100 bar, where p is the vapor pressure of the reaction mixture at the reaction temperature, and
d) 8 to 30 moles of the ketone of the formula III are used per mole of aldehyde of the formula II.

2. A process for the preparation of unsaturated ketones of the general formula I as claimed in claim 1, wherein the temperature is kept at from 25 to 75°C above the boiling point of the lowest-boiling component during the reaction.

**Revendications**

1. Procédé pour la préparation de cétones insaturées de formules générales Ia et/ou Ib

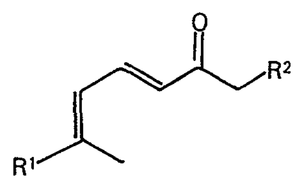
(I a)

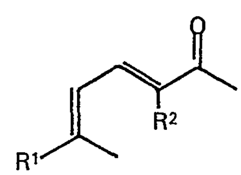
(I b)

dans lesquelles le radical $R^1$ peut avoir la signification suivante:

$-CH_3$; 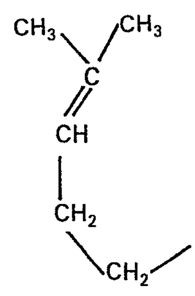 ou 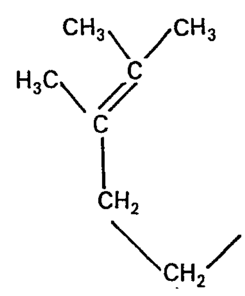

et le radical $R^2$ est mis pour H ou $-CH_3$, par réaction d'un aldéhyde de formule générale II

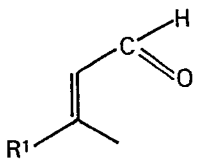

(II)

avec un excès molaire d'une cétone de formule générale III

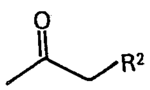

(III)

à température élevée et en présence d'une lessive alcaline aqueuse, caractérisé en ce que
a) l'on effectue la réaction en mélangeant tous les partenaires réactionnels de manière suffisamment énergique pour que le mélange réactionnel à deux phases ne se sépare pas et que le diamètre des gouttes dans ce mélange soit égal ou inférieur à 1 mm,
b) l'on maintient cependant une température qui se situe 10 à 120°C au-dessus du point d'ébullition du partenaire réactionnel dont le point d'ébullition est le plus bas à la pression normale,
c) l'on maintient une pression de p à 100 bar, p étant la pression de vapeur du mélange réactionnel à la température de réaction et
d) l'on met en réaction la cétone de formule III dans des proportions de 8 à 30 mol par mol d'aldéhyde de formule II.

2. Procédé pour la préparation de cétones insaturées de formule générale I selon la revendication 1, caractérisé en ce qu'on maintient, pendant la réaction, une température qui se situe 25 à 75°C au-dessus du point d'ébullition du partenaire réactionnel dont le point d'ébullition est le plus bas.